# EUROPEAN PATENT APPLICATION

(11) **EP 2 116 236 A1**
(43) Date of publication of application: **11.11.2009**
(21) Application number: 08290391.5
(22) Date of filing: 21.04.2008
(51) Int. Cl.: A61K 31/155, A61K 31/496, A61P 39/06, A61P 25/00

(54) **Bisbenzamidine derivatives for use as antioxidant**

(71) Applicant: Université de Mons-Hainaut, 7000 Mons (BE); INSERM, 75654 Paris Cédex 13 (FR)
(72) Inventor: Vanden Eynde, Jean, Jacques, 7000 Mons (BE); Stanicki, Dimitri, 6141 Forchies-La-Marche (BE); Maurois, Pierre, 59790 Ronchin (FR); Vamecq, Joseph, 7030 Saint-Symphorien (BE); Gressens, Pierre, 95470 St-Witz (FR)
(74) Representative: Brants, Johan P.E.

(57) **Abstract**

The invention is directed to the use of bisbenzamidine compounds of Formula (I) as antioxidant and as neuroprotective agents, 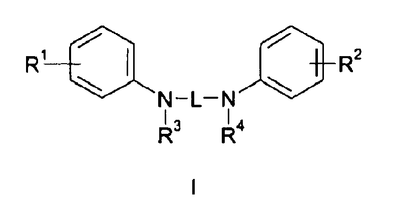 wherein R¹, R², R³, R⁴ and L have the meaning defined in the claims.

## Description

### Field of the invention

The invention is directed to the use of bisbenzamidine derivatives as antioxidant and as neuroprotective agents.

### Background of the invention

Oxidative stress has been proposed as a pathogenic mechanism in Alzheimer's disease (AD) (P. F. Good et al., Am. J. Pathol. (1996) 149:21). Oxidative stress may also contribute to neuronal degeneration and death in disorders ranging from ischemic stroke to Alzheimer's and Parkinson's to age related macular degeneration to amyotrophic lateral sclerosis (M. P. Mattson et al., J. Neurosci. Res. (1997) 49:681), disorders in which nitric oxide, via peroxynitrite, plays a key role.

The link between oxidative stress and acute or chronic neurodegenerative diseases is widely documented in the literature as well as therapeutic approaches to treat these diseases by an antioxidant. Several articles described works that clearly demonstrated the occurrence of oxidative stress in different neurodegenerative diseases (Barber SC, et al. Biochim Biophys Acta 1762 (2006) 1051-1067; Barone FC & Kilgore KS. Clin Neurosci Res 6 (2006) 329-356; Cui K, et al. Prog Neuro-Psychoph 28 (2004) 771- 799; Koutsilieria E, et al. Parkinsonism Relat D 8 (2002) 401-406; Maguire-Zeissa KA, et al. Mol Brain Res 134 (2005) 18-23, Mariani E, et al. J Chromatogr B, 827 (2005) 65-75; Milhavet O & Lehmann S. Brain Research Reviews 38 (2002) 328-339; Pappolla MA, et al. Free Rad Biol Med, Vol. 33, No. 2, pp. 173-181, 2002; Trushina E & McMurray CT. Neuroscience 145 (2007) 1233-1248), including Alzheimer's disease, Parkinson's disease, amyotrophic lateral sclerosis , ischemic vascular disease, or prion (encephalitis). On the other hand, the benefits of antioxidant approach in cardiovascular disease and the development of atherosclerosis are well documented (Kaliora AC, et al. Atherosclerosis 187 (2006) 1-17). The oxidative stress also contributes to the pathophysiology of other human diseases (Valko M, et al. Int J Biochem Cell Biol 39 (2007) 44-84) wherein it is also considered as a therapeutic target.

Several strategies for conferring neuroprotection have been developed which target the complex neurochemical processes which follow neuronal malfunction. However diseases of the central nervous system such as epilepsy and neurodegenerative diseases have proved difficult to treat.

Epilepsy is a condition in which a person has recurrent seizures due to a chronic, underlying process. Epilepsy refers to a clinical phenomenon rather than a single disease entity, since there are many forms and causes of epilepsy. Using a definition of epilepsy as two or more unprovoked seizures, the incidence of epilepsy is estimated at approximately 0.3 to 0.5 percent in different populations throughout the world, with the prevalence of epilepsy estimated at 5 to 10 people per 1000.

Accepted drugs for the treatment of epilepsy are anticonvulsant agents or, more properly termed, anti-epileptic drugs (AEDs), wherein the term "anti- epileptic" is synonymous with "anti-seizure" or "anti-ictogenic". Despite the numerous drugs available for the treatment of epilepsy (i.e., through suppression of ictus epilepticus, i.e., the convulsions associated with epileptic seizures) and other analogous seizure-related disorders, there are no generally accepted drugs for treating, preventing, reversing, arresting or inhibiting the underlying process of epileptogenesis that may be etiologic in many devastating neurological and psychiatric disorders such as epilepsy and other disorders including Bipolar Disorder. In addition, about a quarter of the patients do not respond to the anti-epileptic drugs currently available. Alternative approaches for drug-resistant epilepsy include the implantation of a stimulator of the vagus nerve, or a ketogenic diet. In case of failure of these alternative approaches, drug-resistant epilepsy requires surgery on the brain.

There is a need to develop new antioxidants suitable for the treatment of oxidative stress mediated diseases. There is also a need to find new neuroprotective and/or anti-epileptic drugs capable of reducing the number of drug-resistant epilepsy, and thereby the number of patients undergoing surgery.

### Summary of the invention

The present inventors have found that bisbenzamidine derivatives of Formula (I) are potent antioxidant and can be particularly useful for the prevention or treatment of oxidative stress related diseases. The compounds are also useful for neuroprotection, as anticonvulsant and for the treatment of wounds.

The invention is therefore directed to a new medical use of, and method of prevention and/or treatment using, bisbenzamidine derivatives of Formula (I) as oxygen radical scavengers, or antioxidants, for protection of vital organs, particularly the central nervous system including the brain, from oxidative damage.

In an embodiment, the present invention provides a new use for such bisbenzamidine compounds of Formula (I) for making pharmaceutical compositions useful in prevention of organ reperfusion injury including related acute inflammation, particularly neuroprotection, that is, protection of the central nervous system from traumatic and post-traumatic injury associated with stroke, e.g., prevention of stroke and neurotrauma, and reduction of morbidity resulting from the sequelae of stroke.

The compounds of Formula (I) for use in the invention have the particularity of crossing the blood-brain barrier, and have therapeutic potential in various diseases affecting the central nervous system. These diseases include in a general way the different epileptic syndromes and neurodegenerative diseases, as well as brain diseases wherein therapy involves crossing of the blood-brain barrier.

The present invention relates to compounds of Formula (I) or stereoisomers, tautomers, racemics, metabolites, pro- or predrugs, salts, hydrates, or solvates thereof, which are useful as antioxidant, 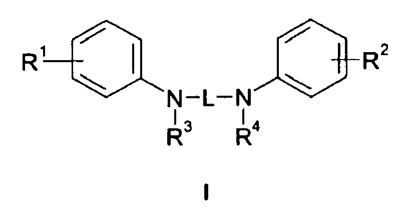 wherein
L represents a C₁₋₁₂alkylene, C₁₋₆alkylenoxyC₁₋₆alkylene, or C₁₋₆alkyleneaminoC₁₋₆alkylene;
each R¹ and R² is independently selected form hydrogen, or a group of Formula (a1), (a2), or (a3); 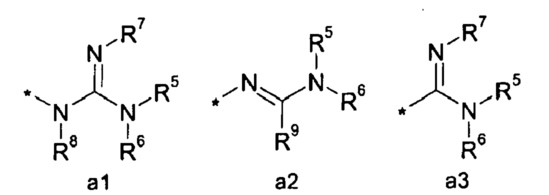 wherein each R⁵, R⁶, R⁷, R⁸ or R⁹ is independently hydrogen or a C₁₋₆alkyl; and

R³ and R⁴ are each independently selected from hydrogen, C₁₋₁₂alkyl, C₁₋₆alkyloxyC₁₋₆alkyl, and C₁₋₆alkylaminoC₁₋₆alkyl, or R³ and R⁴ together with the nitrogen atoms to which they are attached form a five-, six-, or seven-membered heterocyclic ring optionally containing at least one additional heteroatom selected from oxygen, or nitrogen.

Unless a context dictates otherwise, asterisks are used herein to indicate the point at which a mono- or bivalent group depicted herein is connected to the structure to which it relates and of which the group forms part.

The present invention also provides a method of treating or preventing an oxidative stress-related disease, including a neurological disorder, a cardiovascular disorder, or wound, in a subject in need thereof comprising administering an effective amount of a compound of Formula (I) to said subject.

The inventors have shown that the compounds had relatively low efficiency in the maximal electroshock seizure (MES) test (60Hz) and the test pentylenetetrazol-induced seizures, whereas these compounds are active in the test of audiogenic seizure in magnesium deficient animals, with the effective dose 50 (i.e. dose protecting 50% of the tested animals) of the most active compound being in the order of 6 mg compound/kg body weight. These compounds are also active in the electroshock test at 6 Hz. In view of a different mode of action compared to conventional anti-epileptic medication, the compounds for use in the present invention provide therefore a potentially promising alternative treatment for drug-resistant epilepsy, the seizures of which the resolution is not achieved by targeting mechanisms covered by the conventional anti-epileptic drugs.

In addition to their effective anti-convulsant effects in experimental models, and without being bound to any theory, it is believed that the present compound have the advantage of acting according to mechanisms distinct from conventional mechanisms such as GABAergic activity or blocking sodium channels.

With regard to the neurodegenerative diseases, the compounds for use in the invention decrease neuronal and/or astrocyte excitability which is on the one hand in favor of neuroprotection and on the other hand unfavorable to the excitotoxic cascade, a biological event amplifying the extent of brain damage.

The compounds are therefore useful for the prevention or treatment of medical conditions such as acute neurotoxicity (e.g. stroke) and chronic neurotoxicity (e.g. Parkinson's disease, Hungtington's disease, Alzheimer's disease and amyotrophic lateral sclerosis).

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### Detailed description of the invention

The inventors have found that the compounds of Formula (I) are particularly useful as antioxidant, and have potential for the prevention and/or treatment of oxidative stress related diseased.

In an embodiment, the invention provides compounds of Formula (I) for use in the invention, wherein L represents a C₁₋₁₂alkylene, C₁₋₆alkylenoxyC₁₋₆alkylene, or C₁₋₆alkyleneaminoC₁₋₆alkylene; preferably L represents a C₁₋₆alkylene, C₁₋₄alkylenoxyC₁₋₄alkylene, or C₁₋₄alkyleneaminoC₁₋₄alkylene, more preferably L represents a C₁₋₆alkylene, yet more preferably a C₁₋₅alkylene, yet more preferably a C₁₋₄alkylene, yet more preferably a C₁₋₃alkylene, yet more preferably a C₁₋₂alkylene,
each R¹ and R² is independently selected form hydrogen, or a group of Formula (a1), (a2), or (a3); 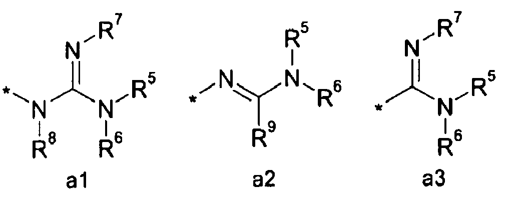 wherein each R⁵, R⁶, R⁷, R⁸ or R⁹ is independently hydrogen or a C₁₋₆alkyl; preferably each R⁵, R⁶, R⁷, R⁸ or R⁹ is independently hydrogen or a C₁₋₄alkyl, preferably each R⁵, R⁶, R⁷, R⁸ or R⁹ is independently hydrogen or a C₁₋₃alkyl, preferably each R⁵, R⁶, R⁷, R⁸ or R⁹ is independently hydrogen or a C₁₋₂alkyl, yet more preferably each R⁵, R⁶, R⁷, R⁸ or R⁹ is independently hydrogen;

R³ and R⁴ are each independently selected from hydrogen, C₁₋₁₂alkyl, C₁₋₆alkyloxyC₁₋₆alkyl, and C₁₋₆alkylaminoC₁₋₆alkyl, preferably R³ is selected from hydrogen, C₁₋₆alkyl, C₁₋₆alkyloxyC₁₋₆alkyl, and C₁₋₆alkylaminoC₁₋₆alkyl, and R⁴ is selected from hydrogen, C₁₋₆alkyl, C₁₋₆alkyloxyC₁₋₆alkyl, and C₁₋₆alkylaminoC₁₋₆alkyl, preferably R³ is selected from hydrogen or C₁₋₆alkyl, and R⁴ is selected from hydrogen or C₁₋₆alkyl, preferably R³ is selected from hydrogen or C₁₋₄alkyl, and R⁴ is selected from hydrogen or C₁₋₄alkyl, for example R³ is selected from hydrogen, methyl, ethyl, *n*-propyl, *iso*-propyl, *n*-butyl, *iso*-butyl, or *tert*-butyl, and R³ is selected from hydrogen, methyl, ethyl, *n*-propyl, *iso*-propyl, *n*-butyl, *iso*-butyl, or *tert*-butyl;
or R³ and R⁴ together with the nitrogen atoms to which they are attached form a five-, six-, or seven-membered heterocyclic ring optionally containing at least one additional heteroatom selected from oxygen, or nitrogen, preferably R³ and R⁴ together with the nitrogen atoms to which they are attached form a five-, six-, or seven-membered heterocyclic ring, preferably R³ and R⁴ together with the nitrogen atoms to which they are attached form a heterocyclic ring selected from imidazolidinyl, imidazolinyl, piperazinyl, or homopiperazinyl.

When describing the compounds of the invention, the terms used are to be construed in accordance with the following definitions, unless a context dictates otherwise:

The term "alkyl" by itself or as part of another substituent refers to a hydrocarbon radical of Formula CₓH₂ₓ₊₁ wherein x is a number greater than or equal to 1. Generally, alkyl groups of this invention comprise from 1 to 20 carbon atoms, more preferably from 1 to 12 carbon atoms, more preferably 1 to 10 carbon atoms, still more preferably 1 to 8 carbon atoms, in particular 1 to 6 carbon atoms, preferably 1 to 4 carbon atoms. Alkyl groups may be linear or branched and may be substituted as indicated herein. When a subscript is used herein following a carbon atom, the subscript refers to the number of carbon atoms that the named group may contain. Thus, for example, C₁₋₁₂alkyl means an alkyl of one to 12 carbon atoms. Examples of alkyl groups are methyl, ethyl, *n*-propyl, *i*-propyl, butyl, and its isomers (e.g. *n*-butyl, *i*-butyl and *t*-butyl); pentyl and its isomers, hexyl and its isomers, heptyl and its isomers, octyl and its isomers, nonyl and its isomers; decyl and its isomers. C₁₋₆alkyl includes all linear, or branched alkyl groups with between 1 and 6 carbon atoms, and thus includes methyl, ethyl, *n*-propyl, *i*-propyl, butyl and its isomers (e.g. *n*-butyl, *i-*butyl and t-butyl); pentyl and its isomers, hexyl and its isomers.

Where alkyl groups as defined are divalent, i.e., with two single bonds for attachment to two other groups, they are termed "alkylene" groups. Generally, alkylene groups of this invention preferably comprise the same number of carbon atoms as their alkyl counterparts. Where an alkylene is present, connectivity to the molecular structure of which it forms part may be through a common carbon atom or different carbon atom, preferably a common carbon atom. To illustrate this applying the asterisk nomenclature of this invention, a C₃ alkylene group may be for example *-CH₂CH₂CH₂-*, *-CH(-CH₂CH₃)-_{*}, or *-CH₂CH(-CH₃)-*.

The term "C₁₋₆alkylenoxyC₁₋₆alkylene" as a group or part of a group represents a group of Formula -R^{a}-O-R^{b}-, wherein R^{a} and R^{b} are each independently a C₁₋₆alkylene as defined herein above.

The term "C₁₋₆alkyleneaminoC₁₋₆alkylene" as a group or part of a group represents a group of Formula -R^{a}-NR^{c}-R^{b}-, wherein R^{a} and R^{b} are each independently a C₁₋₆alkylene as defined herein above, and R^{c} is hydrogen or C₁₋₆alkyl as defined herein above.

The term "C₁₋₆alkyloxyC₁₋₆alkyl" as a group or part of a group represents a group of Formula -R^{a}-O-R^{d}, wherein R^{a} is C₁₋₆alkylene as defined herein above, and R^{d} is C₁₋₆alkyl as defined herein above.

The term "C₁₋₆alkylaminoC₁₋₆alkyl" as a group or part of a group represents a group of Formula -R^{a}-NR^{c}-R^{d}, wherein R^{a} is a C₁₋₆alkylene as defined herein above, R^{c} is hydrogen or C₁₋₆alkyl as defined herein above and R^{d} is C₁₋₆alkyl as defined herein above.

The terms "heterocyclyl" or "heterocyclic" as used herein by itself or as part of another group refer to non-aromatic, fully saturated or partially unsaturated 5 to 7 member monocyclic ring which has at least two nitrogen atom in said ring in at least one carbon atom-containing ring. Said ring may be optionally containing at least one additional heteroatom selected from oxygen, sulfur or nitrogen.

Exemplary heterocyclic groups include imidazolidinyl, imidazolinyl, piperazinyl, and homopiperazinyl.

As used in the specification and the appended claims, the singular forms "a", "an", and "the" also include plural referents unless the context clearly dictates otherwise. By way of example, "a compound" means one compound or more than one compound.

The terms described above and others used in the specification are well understood to those in the art.

Preferred features of the compounds of this invention are now set forth.

In a particular embodiment, the present invention provides the use of a compound of Formula (I) as an antioxidant, wherein L represents a C₁₋₁₂alkylene, and R¹, R², R³ and R⁴ have the same meaning as that defined herein.

In a particular embodiment, the present invention provides the use of a compound of Formula (I) as an antioxidant, wherein each R¹ and R² is independently selected form hydrogen, or a group of Formula (a3), wherein each R⁵ and R⁶ is independently hydrogen or a C₁₋₆alkyl, and L, R³ and R⁴ have the same meaning as that defined herein.

In a particular embodiment, the present invention provides the use of a compound of Formula (I) as an antioxidant, wherein R³ and R⁴ are each independently selected from hydrogen or C₁₋₁₂alkyl, or R³ and R⁴ together with the nitrogen atoms to which they are attached form a five-, six- or seven-membered heterocyclic ring, and R¹, R², and L have the same meaning as that defined herein.

In a particular embodiment, the present invention provides the use of a compound of Formula (I) as an antioxidant, wherein L represents a C₁₋₆alkylene; each R¹ and R² is independently a group of Formula (a3), wherein each R⁵ and R⁶ is independently hydrogen; R³ and R⁴ are each independently selected from hydrogen or C₁₋₆alkyl, or R³ and R⁴ together with the nitrogen atoms to which they are attached form a six-membered heterocyclic ring.

In a particular embodiment, the present invention provides the use of a compound of Formula (I) as an antioxidant, wherein L represents a C₁₋₄alkylene; each R¹ and R² is independently a group of Formula (a3), wherein each R⁵ and R⁶ is independently hydrogen; R³ and R⁴ are each independently selected from hydrogen or C₁₋₆alkyl.

In a particular embodiment, the present invention provides the use of a compound of Formula (I) as an antioxidant, wherein said compound is selected from 4,4'-(piperazine-1,4-diyl)bis(benzenecarboximidamide), 4,4'-( N-methyl 1,2-ethanediyldiimino)bis(benzenecarboximidamide); 4,4'-( N-ethyl 1,2-ethanediyldiimino)bis(benzenecarboximidamide), 4,4'-(1,2-ethanediyldiimino)bisbenzenecarboximidamide; or 4,4'-[1,2-ethanediyl (N,N'-dimethyl bisnitrilo)]bis(benzenecarboximidamide).

It will be clear to the skilled person that the compounds for use in the invention may exist in the form of different isomers and/or tautomers, including but not limited to geometrical isomers, conformational isomers, E/Z-isomers, stereochemical isomers (i.e. enantiomers and diastereoisomers) and isomers that correspond to the presence of the same substituents on different positions of the rings present in the compounds of the invention. All such possible isomers, tautomers and mixtures thereof are included within the scope of the invention.

The compounds may be in the form of pharmaceutically and/or veterinary acceptable salts, as generally described below. Some preferred, but non-limiting examples of suitable pharmaceutically acceptable organic and/or inorganic acids are as hydrochloric acid, hydrobromic acid, sulfuric acid, nitric acid, acetic acid and citric acid, as well as other pharmaceutically acceptable acids known per se (for which reference is made to the prior art referred to below).

The compounds contain a hydrogen-donating heteroatom (e.g. NH), therefore the invention also covers salts and/or isomers formed by transfer of said hydrogen atom to a basic group or atom within the molecule.

The invention also generally covers the use of all pharmaceutically acceptable predrugs and prodrugs of the compounds of Formula (I) for which general reference is made to the prior art cited hereinbelow.

The term "pro-drug" as used herein means the pharmacologically acceptable derivatives such as esters, amides, and phosphates, such that the resulting in vivo biotransformation product of the derivative is the active drug. The reference by Goodman and Gilman (The Pharmacological Basis of Therapeutics, 8th Ed, McGraw-Hill, Int. Ed. 1992, "Biotransformation of Drugs", p 13-15) describing pro-drugs generally is hereby incorporated. Pro-drugs of the compounds can be prepared by modifying functional groups present in said component in such a way that the modifications are cleaved, either in routine manipulation or in vivo, to the parent component. Typical examples of pro-drugs are described for instance in WO 99/33795, WO 99/33815, WO 99/33793, and WO 99/33792 all incorporated herein by reference. Pro-drugs are characterized by increased bio-availability and are readily metabolized into the active inhibitors in vivo. The term "pre-drug", as used herein, means any compound that will be modified to form a drug species, wherein the modification may take place either inside or outside of the body, and either before or after the pre-drug reaches the area of the body where administration of the drug is indicated.

For pharmaceutical use, the compounds may be used as a free acid or base, and/or in the form of a pharmaceutically acceptable acid-addition and/or base-addition salt (e.g. obtained with non-toxic organic or inorganic acid or base), in the form of a hydrate, solvate and/or complex, and/or in the form or a pro-drug or pre-drug, such as an ester. As used herein and unless otherwise stated, the term "solvate" includes any combination which may be formed by a compound of this invention with a suitable inorganic solvent (e.g. hydrates) or organic solvent, such as but not limited to alcohols, ketones, esters and the like. Such salts, hydrates, solvates, etc. and the preparation thereof will be clear to the skilled person; reference is for instance made to the salts, hydrates, solvates, etc. described in US-A-6,372,778, US-A-6,369,086, US-A-6,369,087 and US-A-6,372,733.

The pharmaceutically acceptable salts of the compounds, i.e. in the form of water-, oil-soluble, or dispersible products, include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalene-sulfonate, nicotinate, oxalate, palmoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. In addition, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

The present compounds have the advantage of crossing the blood-brain barrier into the central nervous system, a crucial step in delivering drugs to diseased brains.

The present invention provides compounds of Formula (I) for use as antioxidant, in particular for use in the prevention or treatment of oxidative stress-related diseases.

The present invention also provides the use of a compound of Formula (I) for the preparation of a medicament for the treatment of oxidative stress-related diseases.

In an embodiment, the oxidative stress-related disease is a neurological disorder, with the proviso that in said compound, R³ and R⁴ together with the nitrogen atoms to which they are attached do not form a five-, six-, or seven-membered heterocyclic ring optionally containing at least one additional heteroatom selected from oxygen, or nitrogen. Preferably, the neurological disorder is selected from the group consisting of ischemic brain injury, trauma, epilepsy, arthritis, respiratory distress, Parkinson's disease, Huntington's disease, Alzheimer's disease amyotrophic lateral sclerosis, retinal degeneration, tauopathy, and dementia.

In another embodiment, the oxidative stress-related disease is a wound, preferably a slow healing wound.

The invention provides a method of treating or preventing an oxidative stress- related disease, including a neurological disorder, a cardiovascular disorder, or wound, in a patent in need thereof comprising administering an effective amount of a compound of Formula (I) to the patient.

The present compounds of Formula (I) are particularly useful for neuroprotection.

Patients with injury or damage of any kind to the central (CNS) or peripheral (PNS) nervous system including the retina may benefit from these compounds. This nervous system injury may take the form of an abrupt insult or an acute injury to the nervous system as in, for example, acute neurodegenerative disorders including, but not limited to; acute injury, hypoxia- ischemia or the combination thereof resulting in neuronal cell death or compromise. Acute injury includes, but is not limited to, Traumatic Brain Injury (TBI) including, closed, blunt or penetrating brain trauma, focal brain trauma, diffuse brain damage, spinal cord injury, intracranial or intravertebral lesions including, but not limited to, contusion, penetration, shear, compression or laceration lesions of the spinal cord or whiplash shaken infant syndrome.

In addition, deprivation of oxygen or blood supply in general can cause acute injury as in hypoxia and/or ischemia including, but is not limited to, cerebrovascular insufficiency, cerebral ischemia or cerebral infarction including cerebral ischemia or infarctions originating from embolic occlusion and thrombosis, retinal ischemia (diabetic or otherwise), glaucoma, retinal degeneration, multiple sclerosis, toxic and ischemic optic neuropathy, reperfusion following acute ischemia, perinatal hypoxic-ischemic injury, cardiac arrest or intracranial hemorrhage of any type (including, but not limited to, epidural, subdural, subarachnoid or intracerebral hemorrhage).

Trauma or injury to tissues of the nervous system may also take the form of more chronic and progressive neurodegenerative disorders, such as those associated with progressive neuronal cell death or compromise over a period of time including, but not limited to, Alzheimer's disease, Pick's disease, diffuse Lewy body disease, progressive supranuclear palsy (Steel-Richardson syndrome), multisystem degeneration (Shy-Drager syndrome), chronic epileptic conditions associated with neurodegeneration, motor neuron diseases (amyotrophic lateral sclerosis), multiple sclerosis, degenerative ataxias, cortical basal degeneration, ALS-Parkinson's-Dementia complex of Guam, subacute sclerosing panencephalitis, Huntington's disease, Parkinson's disease, synucleinopathies (including multiple system atrophy), primary progressive aphasia, striatonigral degeneration, Machado-Joseph disease or spinocerebellar ataxia type 3 and olivopontocerebellar degenerations, bulbar and pseudobulbar palsy, spinal and spinobulbar muscular atrophy (Kennedy's disease), primary lateral sclerosis, familial spastic paraplegia, Werdnig-Hoffmann disease, Kugelberg-Welander disease, Tay-Sach's disease, Sandhoff disease, familial spastic disease, Wohlfart-Kugelberg-Welander disease, spastic paraparesis, progressive multifocal leukoencephalopathy, familial dysautonomia (Riley-Day syndrome) or prion diseases (including, but not limited to Creutzfeld-Jakob disease, Gerstmann-Strussler-Scheinker disease, Kuru disease or fatal familial insomnia).

In addition, trauma and progressive injury to the nervous system can take place in various psychiatric disorders, including but not limited to, progressive, deteriorating forms of Bipolar disorder or Schizoaffective disorder or Schizophrenia, Impulse Control disorders, Obsessive Compulsive disorder (OCD), behavioral changes in Temporal Lobe Epilepsy and personality disorders.

The term "neuroprotection" as used herein shall mean; inhibiting, preventing, ameliorating or reducing the severity of the dysfunction, degeneration or death of nerve cells, axons or their supporting cells in the central or peripheral nervous system of a mammal, including a human.

Therefore, the term "a patient in need thereof" as used herein will refer to any patient who currently has or may develop any of the above syndromes or disorders, or any disorder in which the patient's present clinical condition or prognosis could benefit from providing neuroprotection to prevent the; development, extension, worsening or increased resistance to treatment of any neurological or psychiatric disorder.

The term "treating" or "treatment" as used herein, refers to any indicia of success in the prevention or amelioration of an injury, pathology or condition, including any objective or subjective parameter such as abatement; remission; diminishing of symptoms or making the injury, pathology, or condition more tolerable to the patient; slowing in the rate of degeneration or decline; making the final point of degeneration less debilitating; or improving a subject's physical or mental well-being. The treatment or amelioration of symptoms can be based on objective or subjective parameters; including the results of a physical examination, neurological examination, and/or psychiatric evaluations.

The term "therapeutic effect" as used herein, refers to the effective provision of antioxidant effects to prevent or minimize the death or damage or dysfunction of the cells of the patient's central or peripheral nervous system or to improve the healing of slow healing of wounds.

Enhancing wound healing includes, but is not limited to, acceleration of the wound healing process by decreasing oxidative stress upon administration of a compound of Formula (I) compared to a control. Other mechanisms of wound healing are also contemplated. In an embodiment of this method, the compound of Formula (I), or a composition thereof, is preferably administered topically to the wound. Wounds include cuts, abrasions, blemishes, ulcers, and burns.

The term "a therapeutically effective amount" as used herein means a sufficient amount of one or more of the compounds of Formula (I) to produce a therapeutic effect, as defined above, in a subject or patient in need of such treatment.

The terms "subject" or "patient" are used herein interchangeably and as used herein mean any mammal including but not limited to human beings including a human patient or subject to which the compounds can be administered. The term mammals include human patients and non- human primates, as well as experimental animals such as rabbits, rats, and mice, and other animals. I

Generally, the compounds may be formulated as a pharmaceutical preparation or pharmaceutical composition comprising at least one compound of Formula (I) and at least one pharmaceutically acceptable carrier, diluent or excipient and/or adjuvant, and optionally one or more further pharmaceutically active compounds.

By means of non-limiting examples, such a formulation may be in a form suitable for oral administration, for parenteral administration (such as by intravenous, intramuscular or subcutaneous injection or intravenous infusion), for topical administration (including ocular), for administration by inhalation, by a skin patch, by an implant, by a suppository, etc.. Such suitable administration forms - which may be solid, semi-solid or liquid, depending on the manner of administration - as well as methods and carriers, diluents and excipients for use in the preparation thereof, will be clear to the skilled person; reference is again made to for instance US-A-6,372,778, US-A-6,369,086, US-A-6,369,087 and US-A-6,372,733, as well as to the standard handbooks, such as the latest edition of Remington's Pharmaceutical Sciences.

Some non-limiting examples of such preparations include tablets, pills, powders, lozenges, sachets, cachets, elixirs, suspensions, emulsions, solutions, syrups, aerosols, ointments, creams, lotions, soft and hard gelatin capsules, suppositories, eye drops, sterile injectable solutions and sterile packaged powders (which are usually reconstituted prior to use) for administration as a bolus and/or for continuous administration, which may be formulated with carriers, excipients, and diluents that are suitable per se for such formulations, such as lactose, dextrose, sucrose, sorbitol, mannitol, starches, gum acacia, calcium phosphate, alginates, tragacanth, gelatin, calcium silicate, microcrystalline cellulose, polyvinylpyrrolidone, polyethylene glycol, cellulose, (sterile) water, methylcellulose, methyl- and propylhydroxybenzoates, talc, magnesium stearate, edible oils, vegetable oils and mineral oils or suitable mixtures thereof. The formulations can optionally contain other pharmaceutically active substances (which may or may not lead to a synergistic effect with the compounds of the invention) and other substances that are commonly used in pharmaceutical formulations, such as lubricating agents, wetting agents, emulsifying and suspending agents, dispersing agents, desintegrants, bulking agents, fillers, preserving agents, sweetening agents, flavoring agents, flow regulators, release agents, etc.. The compositions may also be formulated so as to provide rapid, sustained or delayed release of the active compound(s) contained therein, for example using liposomes or hydrophilic polymeric matrices based on natural gels or synthetic polymers. In order to enhance the solubility and/or the stability of the compounds of a pharmaceutical composition according to the invention, it can be advantageous to employ α-, β- or γ-cyclodextrins or their derivatives. An interesting way of formulating the compounds in combination with a cyclodextrin or a derivative thereof has been described in EP-A-721,331. In particular, the present invention encompasses a pharmaceutical composition comprising an effective amount of a compound according to the invention with a pharmaceutically acceptable cyclodextrin.

The preparations may be prepared in a manner known per se, which usually involves mixing at least one compound of Formula (I) with the one or more pharmaceutically acceptable carriers, and, if desired, in combination with other pharmaceutical active compounds, when necessary under aseptic conditions. Reference is again made to US-A-6,372,778, US-A-6,369,086, US-A-6,369,087 and US-A-6,372,733 and the further prior art mentioned above, as well as to the standard handbooks, such as the latest edition of Remington's Pharmaceutical Sciences.

The compounds can be administered by a variety of routes including the oral, rectal, ocular, transdermal, subcutaneous, intravenous, intramuscular or intranasal routes, depending mainly on the specific preparation used and the condition to be treated or prevented, and with oral and intravenous administration usually being preferred. The at least one compound of Formula (I) will generally be administered in an "effective amount", by which is meant any amount of a compound of the Formula (I) that, upon suitable administration, is sufficient to achieve the desired therapeutic or prophylactic effect in the individual to which it is administered. Usually, depending on the condition to be prevented or treated and the route of administration, such an effective amount will usually be between 0.01 to 1000 mg per kilogram body weight of the patient per day, more often between 0.1 and 500 mg, such as between 1 and 250 mg, for example about 5, 10, 20, 50, 100, 150, 200 or 250 mg, per kilogram body weight of the patient per day, which may be administered as a single daily dose, divided over one or more daily doses, or essentially continuously, e.g. using a drip infusion. The amount(s) to be administered, the route of administration and the further treatment regimen may be determined by the treating clinician, depending on factors such as the age, gender and general condition of the patient and the nature and severity of the disease/symptoms to be treated. Reference is again made to US-A-6,372,778, US-A-6,369,086, US-A-6,369,087 and US-A-6,372,733 and the further prior art mentioned above, as well as to the standard handbooks, such as the latest edition of Remington's Pharmaceutical Sciences.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

The invention will now be illustrated by means of the following synthetic and biological examples, which do not limit the scope of the invention in any way.

### Examples

### 1-Preparation of the compounds for use in the present invention

The compounds can be prepared following the procedure described in Scheme 1.

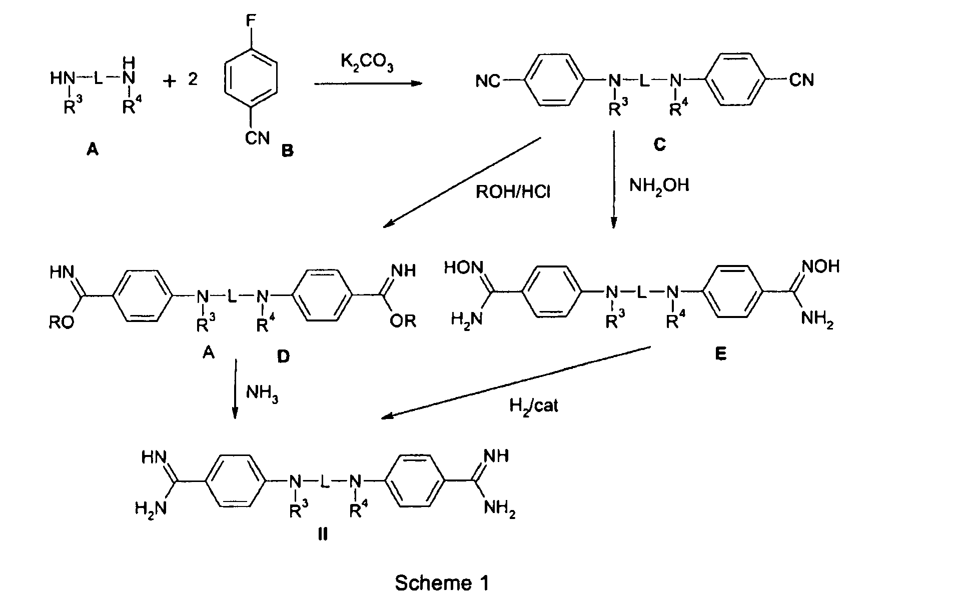

The first step consisted in the synthesis of bisbenzonitrile intermediates **(C)** by reaction of a diamine **(A)** with 4-fluorobenzonitrile **(B)** in boiling *N*,*N*-dimethylacetamide in the presence of a base such as K₂CO₃.

Conversion of the nitrile functions of **(C)** into amidines in compound **(II)** was achieved either by the Pinner reaction which comprises the conversion into the imidate **(D)** by action of an alcohol in the presence of hydrochloric acid and further reaction with an amine, or through formation and reduction of the corresponding amidoximes **(E).** The synthetic route was dependent on the solubility of the dinitrile in the presence of hydrochloric acid.

### 1.1- Synthesis of the bisbenzonitrile intermediates

The benzonitrile intermediates were prepared following the protocol A described hereunder;

*Protocol A*: A mixture of a diamine (25 mmol), 4-fluorobenzonitrile (55 mmol), and potassium carbonate (50 mmol) in DMA (25 ml) was heated under reflux for 9 h. After cooling, the reaction mixture was poured into cold water, the solid was filtered and successively washed with water and ethanol.

### Intermediate I1: 4,4'-(piperazine-1,4-diyl)bisbenzonitrile

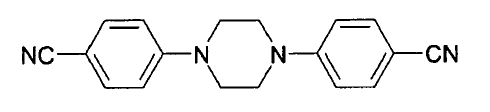

M.p.: 271-273 °C
IR (KBr): v (cm⁻¹) : 3098 (CH arom.), 2963 (CH alip), 2842 (CH alip), 2215 (C=N), 1599, 1513,1177,824
¹H RMN (DMSO *d*₆): δ (ppm) : 7.6 (d, 4 H, Ar adjacent to the nitrile group, J = 9 Hz); 7.0 (d, 4 H, Ar adjacent to the piperazine ring, J = 9 Hz); 3.5 (s, 8 H, piperazine protons) ppm.
Yield : 83%
Literature: Berg, S. S, "Search for Chemotherapeutic Amidines - (XVII).alpha.,.omega.-bis(p-amidinoanilino)alkanes" J. Chem. Soc., 1960, 5172-6.

### Intermediate I2: 4,4'-(1,2-ethanediyldiimino)bis(benzonitrile)

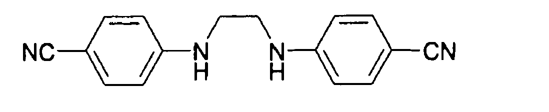

M.p.: 207-210 °C
IR (KBr): v (cm⁻¹) : 3368 (N-H) ; 2211 (C≡N) ; 1604, 1528, 1303, 1283
¹H RMN (DMSO *d₆*) δ (ppm) : 7,5 (4H, d, J = 9Hz, H², H⁶, H^{2'} et H⁶) ; 6,8 (2H, s large, N-H) ; 6,6 (4H, d, J = 9Hz, H³, H⁵, H^{3'} et H^{5'}) ; 3,3 (4H, s, CH₂-NH)
HRMS (ESI-ToF) MH⁺ C₁₆H₁₅N₄: exp.: m/z 263,1296; Calc.: m/z 263,1297
Yield: 70%

### Intermediate 13: 4,4'-[1,2-ethanediyl (N,N-dimethyl bisnitrilo)]bisbenzonitrile

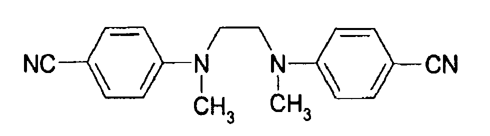

M.p.: 204-206 °C
IR (KBr): v (cm⁻¹) : 2208 (C≡N), 1604, 1520, 1387, 1177
¹H RMN (DMSO *d₆*): δ (ppm): 7,5 (4H, d, J = 8 Hz, H³, H⁵, H^{3'} et H^{5'}) ; 6,7 (4H, d, J = 8 Hz, H², H⁶, H^{2'} et H^{6'}) ; 3,6 (4H, s, CH₃-N-CH₂) ; 2,9 (6H, s, CH₃-N)
HRMS (ESI-ToF) MH⁴ C₁₈H₁₉N₄ : exp.: m/z 291,1613; Calc.: m/z 291,1610
Yield: 68%

### Intermediate I4: 4,4'-(N-methyl 1,2-ethanediyldiimino)bisbenzonitrile

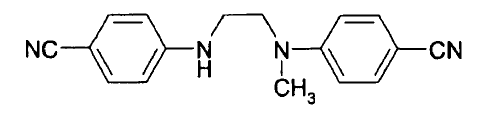

M. p.: 150-152 °C
IR (KBr): v (cm⁻¹): 3388 (N-H), 2214 (C≡N), 1603, 1521, 1386, 1181
¹H RMN (DMSO *d₆*) : δ (ppm) : 7,5 (2H, d, J = 8 Hz, H^{2 (2')} et H^{6 (6')}) ;7,4 (2H, d, J = 8Hz, H^{2 (2')} et H^{6 (6')}) ; 6,8 (1 H, s large, N-H) ; 6,8 (2H, d, J = 8 Hz, H^{3 (3')} et H^{5 (5')} ) ; 6,6 (2H, d, J=8 Hz, H^{3 (3')} et H^{5 (5')}) ; 3,6 (2H, t, J = 6 Hz, NH-CH₂) ; 3,3 (2H, t, J = 6 Hz, Met-N-CH₂) ; 3,0 (3H, s, CH₃-N-CH₂)
HRMS (ESI-ToF) MH⁺ C₁₇H₁₇N₄ : exp.: m/z 277,1453; Calc.: m/z 277,1453
Yield: 71%

### Intermediate I5: 4,4'-(N-ethy/ 1,2-ethanediyldiimino)bisbenzonitrile

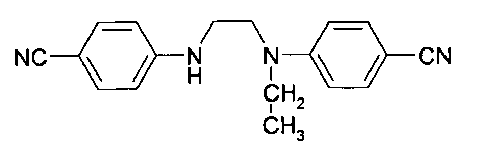

M.p.: 144-146 °C
IR (KBr): v (cm⁻¹): 3380 (N-H), 2211 (C≡N), 1602, 1522, 1178
¹H RMN (DMSO *d*₆) δ (ppm) : 7,6 (2H, d, J = 9 Hz, H^{2 (2')} et H^{6 (6')} ) ; 7,5 (2H, d, J = 9Hz, H^{2 (2')} et H^{6 (6')} ; 6,8 (1 H, s large, N-H) ; 6,8 (2H, d, J = 9 Hz, H^{3 (3')} et H^{5(5')} ) ; 6,5 (2H, d, J=9Hz, H^{3 (3')} et H^{5 (5')}) ; 3,5 (2H, t, J = 6Hz, NH-CH₂) ; 3,4 (2H, q, J = 7 Hz, N-CH₂-CH₃) ; 3,3 (2H, t, J = 6 Hz, Et-N-CH₂) ; 1,1 (3H, t, J = 7 Hz, CH₃-CH₂)
HRMS (ESI-ToF) MH⁺ C₁₈H₁₉N₄ : exp.: m/z 291,1600; Calc. : m/z 291,1610
Yield: 23%

### 1.2- Synthesis of the diamidines

### 2A. By the Pinner reaction following protocol B

*Protocol B:* A mixture of bisbenzonitrile intermediate (3.4 mmol) and methanol (20 ml) in dichloromethane (200 ml) was saturated with gaseous hydrochloric acid. After 4 days at room temperature, the solvents were evaporated under reduced pressure and the solid was washed with ether. The imidate was then treated with a methanolic solution of ammonia (7 N, 15 ml) and the mixture was heated under reflux for 1 h. After cooling the precipitate was filtered and washed with alcohol.

### Compound 1: 4,4'-(piperazine-1,4-diyl)bis(benzenecarboximidamide)

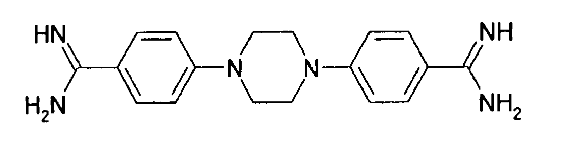

M.p.: > 300 °C
IR (KBr) : ν (cm⁻¹) : 3099 (NH); 1660; 1606; 1487; 1398; 1034
¹H RMN (DMSO *d₆*): δ (ppm): 9.0 (br, 4 H, amidine); 8.7 (br, 4H, amidine); 7.8 (d, 4 H, Ar adjacent to the amidine group, J = 9 Hz); 7.1 (d, 4 H, Ar adjacent to the piperazine ring, J = 9 Hz); 3.6 (s, 8 H, piperazine protons)
Yield: 55 %
Literature: Tao, B.; Huang, T. L.; Zhang, Q.; Jackson, L.; Queener, S. F.; Donkor, I. O., "Synthesis and anti-pneumocystis carinii activity of conformationally restricted analogues of pentamidine," Eur. J. Med. Chem., 1999, 34, 531-538.

### Compound 4: 4,4'-( N-methyl 1,2-ethanediyldiimino)bis(benzenecarboximidamide)

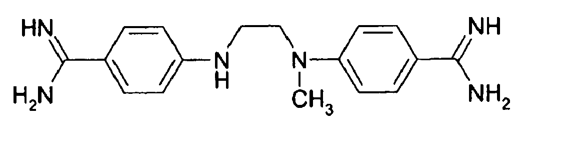

M.p.: > 300 °C
IR (KBr): v (cm⁻¹): 3329, 3143, 1661, 1608, 1495
¹H RMN (DMSO *d₆*): δ (ppm) : 9,1 (2H, s, N-H amidine) ; 8,9 (2H, s, N-H amidine) ; 8,8 (2H, s, N-H amidine) ; 8,7 (2H, s, N-H amidine) ; 7,8 (2H, d, J = 9Hz, H^{2 (2')} et H^{6 (6')}) ; 7,7 (2H, d, J = 9 Hz, H^{2 (2')} et H^{6 (6')}) ; 7,1 (1H, t large, N-H); 6,8 (2H, d, J = 9 Hz, H^{3 (3')} et H^{5 (5')} : 6,7 (2H, d, J = 9 Hz, H^{5 (5')} et H^{3 (3')}) ; 3,6 (2H, t, J = 5 Hz, CH₂-NH); 3,4 (2H, t, J = 5 Hz, Et-N-CH₂) ; 3,0 (3H, s, CH₃-N-CH₂)
HRMS (ESI-ToF) MH⁺ C₁₇H₂₃N₆ : exp.: m/z 311,1970; Calc.: m/z 311,1984
Yield: 36%

### Compound 5: 4,4'-(N-ethyl-1,2-ethanediyldiimino)bis(benzenecarboximidamide)

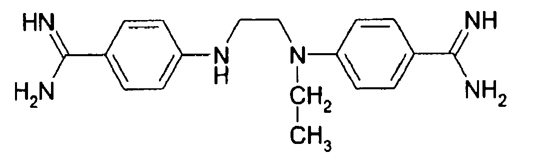

M.p.: > 300 °C
IR (KBr): v (cm⁻¹): 3317, 3136, 1663, 1605, 1494
¹H RMN (DMSO *d₆*) : δ (ppm) : 9,1 (2H, s, N-H amidine) ; 8,9 (2H, s, N-H amidine) ; 8,8 (2H, s, N-H amidine) ; 8,7 (2H, s, N-H amidine) ; 7,8 (2H, d, J = 9Hz, H^{2 (2')} et H^{6 (6')}) ; 7,7 (2H, d, J = 9 Hz, H^{2 (2' )} et H^{6 (6')}) ; 7,1 (1H, t large, N-H) ; 6,8 (2H, d, J = 9 Hz, H^{3 (3')} et H^{5 (5')}) ; 6,7 (2H, d, J = 9 Hz, H^{5 (5')} et H^{3 (3')}) ; 3,6 (2H, t, J = 8 Hz, CH₂-NH) ; 3,5 (2H, q, J = 7Hz, CH₃-CH₂-N) ; 3,4 (2H, t, J = 8 Hz, Et-N-CH₂) ; 1,1 (3H, t, J = 7Hz, CH₃-CH₂)
HRMS (ESI-ToF) MH⁺ C₁₈H₂₅N₆ : exp.: m/z 325,2136; Calc.: m/z 325,2141
Yield: 33%

### 2B. By reduction of amidoximes following protocol C

*Protocol C*: A mixture of hydroxylamine hydrochloride (40 mmol) and a methanolic solution of sodium methoxide (30 %, 40 mmol) in methanol (25 ml) was heated under reflux for 30 min. After cooling the precipitate was filtered and the filtrate was transferred into a round-bottom flask. The bisbenzonitrile (4 mmol) was added and the mixture was heated under reflux for 8 h. After cooling the precipitate was filtered and washed with methanol.

The obtained diamidoxime (2.2 mmol) was dissolved in acetic acid (15 ml). Ammonium formate (25 mmol) and Pd/C 10 % (0.2 g) were successively added. The mixture was heated under reflux for 2 h. After cooling the mixture was filtered on Celite^{®}and the filtrate was concentrated under reduced pressure. The solid was separated, poured into dichloromethane and precipitated as the dihydrochloride salt by addition of gaseous hydrochloric acid.

### 4,4'-(1,2-ethanediyldiimino)bis(N-hydroxy benzenecarboxymidamide)

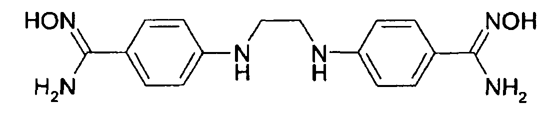

M.p.: 206-209 °C
IR (KBr): v (cm⁻¹): 3489 (NH₂), 3379 (NH₂), 1661, 1608, 1529
¹H RMN (DMSO *d₆*) : δ (ppm) : 9,3 (2H, s, O-H) ; 7,5 (4H, d, J = 9 Hz, H², H⁶, H^{2'}et H^{6'}) ; 6,6 (4H, d, J = 9 Hz, H³, H⁵, H^{3'}et H^{5'}) ; 5,9 (2H, s, N-H) ; 5,6 (4H, s, -NH₂) ; 3,2 (4H, s, NH-CH₂)
HRMS (ESI-ToF) MH⁺ C₁₆H₂₁N₆O₂ : exp.: m/z 329,1728; Calc.: m/z 329,1732
Yield: 87%

### 4,4'-[1,2-ethanediyl (N,N'dimethyl bisnitrilo)]bis(N-hydroxy benzenecarboximidamide)

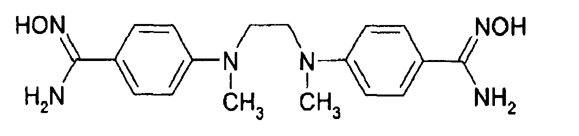

M.p.: 198-202 °C
IR (KBr): v (cm⁻¹): 3496 (NH₂), 3374 (NH₂), 1665, 1609, 1527
¹H RMN (DMSO *d₆*) : δ (ppm) : 9,3 (2H, s, O-H) ; 7,6 (4H, d, J = 9 Hz, H², H⁶, H^{2'}et H^{6'}) ; 6,7 (4H, d, J = 9 Hz, H³, H⁵, H^{3'}et H^{5'}) ; 5,7 (4H, s, -NH₂) ; 3,6 (4H, s, CH₃-N-CH₂) ; 2,9 (6H, s, CH₃-N)
HRMS (ESI-ToF) MH⁺ C₁₈H₂₅N₆O₂ : exp.: m/z 357,2033; calc. : m/z 357,2039
Yield: 90%

### Compound 2: 4,4'-(1,2-ethanediyldiimino)bisbenzenecarboximidamide

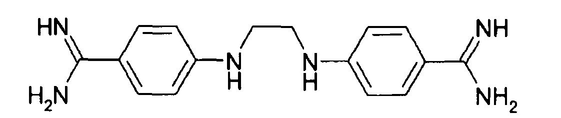

M.p.: > 300 °C
IR (KBr) : v (cm⁻¹) : 3296, 3211, 3122, 1657,1609, 1490, 1460
¹H RMN (DMSO *d₆*) : δ (ppm) : 8,9 (2H, s, N-H amidine) ; 8,7 (4H, s, N-H amidine) ; 7,7 (4H, d, J = 8 Hz, H², H⁶, H^{2'} et H^{6'}) ; 7,1 (2H, s, N-H) ; 6,8 (4H, d, J = 8 Hz, H³, H⁵, H^{3'} et H^{5'}) ; 3,4 (4H, s, CH₃-N-CH₂)
HRMS (ESI-ToF) MH⁺ C₁₆H₂₁N₆ : exp.: m/z 297,1829; Calc.: m/z 297,1828
Yield: 65%

### Compound 3: 4,4'-[1,2-ethanediyl (N,N'-dimethyl bisnitrilo)]bis(benzenecarboximidamide)

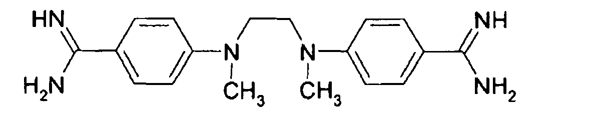

M.p.: > 300 °C
IR (KBr) : v (cm⁻¹) : 3307, 3137, 1664, 1609, 1485
¹H RMN (DMSO *d₆*) : δ (ppm) : 8,9 (2H, s, N-H amidine) ; 8,6 (4H, s, N-H amidine) ; 7,7 (4H, d, J = 8 Hz, H², H⁶, H^{2'} et H^{6'}) ; 6,8 (4H, d, J = 8 Hz, H³, H⁵, H^{3'} et H^{5'}) ; 3,7 (4H, s, CH₃-N-CH₂) ; 3,0 (6H, s, CH₃-N-CH₂)
HRMS (ESI-ToF) MH⁺ C₁₈H₂₅N₆ : exp./ m/z 325,2133; Calc.: m/z 325,2141
Yield: 68%

### 2- Biological activity

### 2-1- Anticonvulsive and neuroprotective activities of the compounds

Compounds showed relatively low efficiency in the maximal electroshock seizure test (60Hz) and in the pentylenetetrazol-induced seizures test, compound 1 and 2 were active in the test of audiogenic seizure in magnesium deficient animals. The effective dose 50 (i.e. dose protecting 50% of the tested animals) of the most active compound was in the order of 6 mg compound/kg body weight. In the electroshock test at 6 Hz, compound 2 was effective at an ED50 between 60 and 100mg/kg.

The tests were performed in rodents as described in J. Vamecq et al. Eur. J. Neurosc. 2003, 18, 1110. The results are shown in Table 1, wherein ScPtz refers to subcutaneous pentylenetetrazol, MES refers to Maximal Electroshock Seizures, MCS refers to Maximal Clonic Seizures, and ND means "not determined". The results are given as ED₅₀ in mg/kg.

**Table 1**

| **Compound** | **audiogenic seizure (mouse ip)** | **chemical seizure (scPtz) (mouse ip)** | **electrical seizure** | |
|---|---|---|---|---|
| | | | **MES (50 Hz) (mouse ip)** | **MCS (6 Hz) (mouse ip)** |
| **1** | 8.6 | inactive up to 300 mg/kg) | inactive up to 30 mg/kg) | ND |
| **2** | 6.9 | inactive up to 300 mg/kg) | Protection (4 animals/5) at 100 mg/kg | ED₅₀ = 48.95 mg/kg |
| **3** | little activity at non toxic doses | inactive up to 300 mg/kg | Protection (1 animal/5) at 100 mg/kg | ND |
| **4** | little activity at non toxic doses | inactive up to 100 mg/kg | inactive up to 100 mg/kg) | ND |
| **5** | little activity at non toxic doses | inactive up to 30 mg/kg) | Protection (3 animals/5) at 30 mg/kg | ND |

Most of the compounds have a protective effect. Compound 2 has a central brain activity and affects the central nervous system and is particularly useful as an anti-convulsing and/or neuroprotective agent. The neuroprotective activity of the compound with respect to the audiogenic seizure in magnesium deficient animals is at least one time higher than the activity measured in conventional animal seizure, thereby showing the potential of said compound as a neuroprotective agent.

### 2-2- Antioxidant activity of the compounds

The compounds were evaluated for their ability to demonstrate superoxide dismutase activity (scavenging activity toward superoxide anion), and a glutathione peroxidase activity (stimulation of the peroxide induced oxidation of glutathione).

### 2-2-1- Superoxide dismutase activity

The compounds have a scavenging activity toward the superoxide anion. This anti-O₂⁻ has been shown by the ability of the compounds to inhibit the pyrogallol auto-oxidation, a process mediated by the superoxide anion.

The pyrogallol auto-oxidation was measured at 420 nm according to the procedure Marklund & Marklund as reported in Vamecq et al. (2003). The incubation medium comprised TRIS saline buffer at pH 7.4 (5 mM TRIS, 50 mM NaCl) with either the vehicle (DMSO) or one of the compounds (final concentration 0.5 mM ) dissolved in DMSO. The reaction was triggered by the addition of pyrogallol (0.750 mM). Preliminary results consist in the measure of the inhibition of pyrogallol auto-oxidation by the different compounds tested.

Each compound has been the subject of three separate measures whose average value was given.

Table 2 shows the results obtained with the five compounds and the result obtained with 4-aminobenzamidine. The latter, unlike the five compounds, does not have any scavenging activity. The results are given as percent inhibition of the rate of pyrogallol auto-oxidation measured during the first two minutes.

**Table 2**

| **experimental conditions** | **Percent inhibition** |
|---|---|
| **Control (vehicle)** | 0% |
| **Compound 1** | 69.4 % |
| **Compound 2** | 36.3 % |
| **Compound 3** | 51.0 % |
| **Compound 4** | 17.5% |
| **Compound 5** | 28.8 % |
| **4-aminobenzaminidine** | < 5% |

### 2-2-2- glutathione peroxidase activity

The glutathione peroxidase activity of the five compounds has also been measured. The activity of glutathione peroxidase compounds refers to the stimulation by the compounds of the peroxide-induced oxidation of reduced glutathione (H₂O₂, t-butyl-hydroperoxide or cumene hydroperoxide). The peroxide-induced oxidation of reduced glutathione was measured by the formation of oxidized glutathione revealed by the addition of glutathione reductase and NADPH. The formation of oxidized glutathione results in the oxidation of NADPH in the presence of glutathione reductase. The NADPH oxidation is then measured and reflects the formation of oxidized glutathione. In view of the absorbance of the compounds, NADPH oxidation was measured by the fluorescent emission at 466 nm when excited at 366 nm, which a corrected quenching.

The glutathione oxidation was measured in the presence of NADPH and glutathione reductase in 1 ml cell by fluorimetry (λ_{excitation} = 366 nm and λₑₘᵢₛₛᵢₒₙ= 466 nm). The cell contained 1 mL of 10 mM phosphate buffer pH 7.4, 0.25 mM NADPH, 10 µl DMSO (or 10 µl 50 mM compound dissolved in DMSO), 2 mM reduced glutathione and two units of glutathione reductase (*Saccharomyces cerevisiae*). The reaction was triggered by the addition of peroxide (5 mM *t*-butyl-hydroperoxide). A stimulation of the rate of peroxide-induced oxidation of glutathione was considered as glutathione peroxidase activity. Each compound was been the subject of 3 separate measures whose average value is given in Table 3.

**Table 3**

| **Experimental conditions** | **Effects of the compounds on the oxidation rate (%)** |
|---|---|
| **Control (vehicle)** | 100 % |
| **Compound 1** | 109 % |
| **Compound 2** | < 100 % |
| **Compound 3** | 197 % |
| **Compound 4** | 107 % |
| **Compound 5** | 105 % |

| | |
|---|---|
| The glutathione peroxidase activity of compound 3 was further investigated. The test was performed a described above. The results are shown in Table 4. | |

**Table 4**

| **Experimental conditions** | **Glutathione peroxidase activity of compound 3** |
|---|---|
| **2 mM reduced Glutathione** | 197 % |
| **5 mM *t*-butyl-hydroperoxide** | |
| **2 mM reduced Glutathione** | 108 % |
| **5 mM hydrogen peroxide** | |
| **2 mM reduced Glutathione** | 117 % |
| **2.5 mM hydrogen peroxide** | |
| **2 mM reduced Glutathione** | 128 % |
| **2.5 mM cumene hydroperoxide** | |

| | |
|---|---|
| While each of the compounds stimulated the initial rate of glutathione oxidation, each of the compounds reduced the intermediate and final oxidation rate. The average glutathione oxidation rate (for example, the *t*-butylhydroperoxide) was modified as reported in Table 3. Table 4 shows that glutathione peroxidase activity of compound 3 had an effect with each of the hydroperoxide tested. | |

In conclusion, compounds 1 to 5 have an antioxidant activity. They have mostly a superoxide dismutase-like activity and can be considered as very active scavengers of the superoxide anion. Some of the compounds have also a glutathione peroxidase-like activity.

This strong antioxidant activity can explain the effects of the compounds on audiogenic seizure of magnesium deficient compounds.

## Claims

1. A compound of Formula (I), or a stereoisomer, tautomer, racemate, metabolite, prodrug, salt, hydrate, or solvate thereof, for use as an antioxidant, 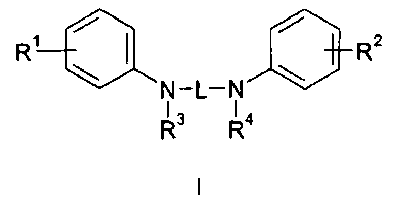 wherein
L represents a C₁₋₁₂alkylene, C₁₋₆alkylenoxyC₁₋₆alkylene, or C₁₋₆alkyleneaminoC₁₋₆alkylene;
each R¹ and R² is independently selected form hydrogen, or a group of Formula (a1), (a2), or (a3); 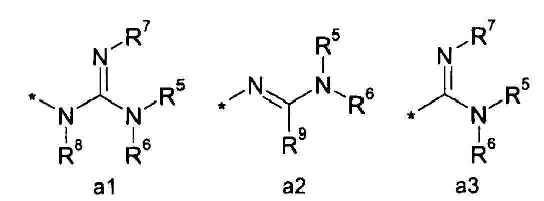 wherein each R⁵, R⁶, R⁷, R⁸ or R⁹ is independently hydrogen or a C₁₋₆alkyl; and
R³ and R⁴ are each independently selected from hydrogen, C₁₋₁₂alkyl, C₁₋₆alkyloxyC₁₋₆alkyl, and C₁₋₆alkylaminoC₁₋₆alkyl, or R³ and R⁴ together with the nitrogen atoms to which they are attached form a five-, six-, or seven-membered heterocyclic ring optionally containing at least one additional heteroatom selected from oxygen, or nitrogen.

2. The compound according to claim 1, wherein L represents a C₁₋₁₂alkylene.

3. The compound according to claim 1 or 2, wherein each R¹ and R² is independently selected form hydrogen, or a group of Formula (a3), wherein each R⁵ and R⁶ is independently hydrogen or a C₁₋₆alkyl.

4. The compound according to any of claims 1 to 3, wherein R³ and R⁴ are each independently selected from hydrogen or C₁₋₁₂alkyl, or R³ and R⁴ together with the nitrogen atoms to which they are attached form a five-, six- or seven-membered heterocyclic ring.

5. The compound according to any of claims 1 to 4, wherein L represents a C₁₋₆alkylene; each R¹ and R² is independently a group of Formula (a3), wherein each R⁵ and R⁶ is independently hydrogen; R³ and R⁴ are each independently selected from hydrogen or C₁₋₆alkyl, or R³ and R⁴ together with the nitrogen atoms to which they are attached form a six-membered heterocyclic ring.

6. The compound according to any of claims 1 to 5, wherein L represents a C₁₋₄alkylene; each R¹ and R² is independently a group of Formula (a3), wherein each R⁵ and R⁶ is independently hydrogen; R³ and R⁴ are each independently selected from hydrogen or C₁₋₆alkyl.

7. The compound according to any of claims 1 to 5, wherein said compound is selected from 4,4'-(piperazine-1,4-diyl)bis(benzenecarboximidamide), 4,4'-( N-methyl 1,2-ethanediyldiimino)bis(benzenecarboximidamide); 4,4'-( N-ethyl 1,2-ethanediyldiimino)bis(benzenecarboximidamide), 4,4'-(1,2-ethanediyldiimino)bisbenzenecarboximidamide ; or 4,4'-[1,2-ethanediyl (N,N'-dimethyl bisnitrilo)]bis(benzenecarboximidamide).

8. The compound according to any of claims 1 to 7, for use in the prevention or treatment of oxidative stress-related diseases.

9. The compound according to claim 8, wherein the oxidative stress-related disease is a neurological disorder, with the proviso that in said compound, R³ and R⁴ together with the nitrogen atoms to which they are attached do not form a five-, six-, or seven-membered heterocyclic ring optionally containing at least one additional heteroatom selected from oxygen, or nitrogen.

10. The compound according to claim 9, wherein the neurological disorder is selected from the group consisting of ischemic brain injury, trauma, epilepsy, arthritis, respiratory distress, Parkinson's disease, Huntington's disease, Alzheimer's disease amyotrophic lateral sclerosis, retinal degeneration, tauopathy, and dementia.

11. The compound according to any of claims 1 to 8, wherein the oxidative stress-related disease is a wound.
